# EUROPEAN PATENT APPLICATION

(11) **EP 1 932 919 A1**
(43) Date of publication of application: **18.06.2008**
(21) Application number: 06126067.5
(22) Date of filing: 13.12.2006
(51) Int. Cl.: C12Q 1/02, C12Q 1/533

(54) **Yeast-based screening method for the identification of compounds inhibiting a human peptidyl-prolyl cis/trans isomerase of the parvulin family**

(71) Applicant: Novartis AG, 4056 Basel (CH)
(72) Inventor: Movva, Rao, 4102 Binningen (CH); Filipuzzi, Ireos, 4058 Basel (CH)
(74) Representative: Bourgarel, Denis Jacques Marie

(57) **Abstract**

The present invention relates to a yeast-based screening method for the identification of compounds selectively and specifically bind to a human peptidyl-prolyl isomerase (PPlase) of the parvulin family.

## Description

### Field of the invention

The present invention relates to a method for the identification of compounds which selectively and specifically bind to a human peptidyl-prolyl isomerase (PPlase) of the parvulin family.

### Background of the invention

Phosphorylation of proteins on serine or threonine residues preceding proline (pSer/Thr-Pro) is a major regulatory mechanism in cell proliferation and transformation. Proline residues exist in two completely distinct *cis* and *trans* conformations. The conformational change following phosphorylation seems to play a pivotal role in regulating the function of affected proteins. The switch in the polypeptide chain backbone is controlled by the peptidyl-prolyl isomerase (PPI) Pin1 in human. The strictly phosphorylation dependent isomerization of phosphorylated pSer/pThr-Pro bonds mediated by Pin1 regulates confirmation and function of a number of phosphoproteins involved in various cellular processes including cell-cycle control, transcription and splicing regulation, DNA replication checkpoint control, DNA damage response, neuronal survival and germ cell development (Wulf et al 2005). Pin1 contains a WW domain which targets the enzyme to its substrates and a PPlase domain inducing the conformational change. Over 35 Pin1-binding proteins have been identified, indicating its regulatory involvement in multiple cellular pathways (Lu 2004).
Studies have suggested that the deregulation of Pin1 may play a pivotal role in various diseases. Notably, the up-regulation of Pin1 may be implicated in certain cancers (reviewed in Lu et al 2006), and the down-regulation of Pin1 may be implicated in Alzheimer's disease (reviewed in Butterfield et al 2006). Pin1 is found to be over-expressed in many human tumors like breast, skin, prostate and colon cancers and its expression level correlates with the grade of the tumor. Furthermore, Pin1 enhances the transformed phenotype of oncogene-transformed mammary epithelial cells. Interestingly, Pin1-deficient mice are found to be resistant to oncogene-induced tumorigenesis (Lu et al 2005). There is therefore a need in the art for human Pin1 inhibitors useful anti-cancer agents.

Peptidyl-prolyl isomerases (PPlase) are highly conserved during evolution and therefore found in bacteria, yeast, plants, fly and mammals. Three families of structurally unrelated prolyl isomerases are known to date comprising cyclophilins, FKBPs and parvulins (reviewed in Galat 2003). Two of the PPlase families represent validated drug targets (reviewed in Cardenas et al 1995), cyclophilin A is known to bind the immunosuppressive drug cyclosporin and FKBP12 binds the immunosuppressive drug FK506. Parvulins are not sensitive to either Cyclosporine A or to FK-506, but can be inhibited by Juglone, a natural product extracted from walnuts. However, Juglone is a covalent modifier and would not be suitable for use as a medicament. There is therefore a need I in the art for alternative inhibitors of Pin1.

### Summary of the invention

The yeast homologue of Pin1, ESS1, is essential for survival and is the only parvulin found in *S. cerevisiae.* An *ESS1* deletion can be rescued by replacing the yeast gene with human (PIN1), fly (dodo) and plant orthologues (DIPar13 and MdPin1). However, in S. *cerevisiae* an *ESS1* deletion can be also be rescued by cyclophilin A (CYP1), a non-parvulin PPlase (Fujimori et al 2001). The present inventors have realized that the functional conservation of Pin1 and the genetic background of parvulins in S. *cerevisiae* allow the establishment of a yeast-based assay system which can be used to set up a differential screen for specific inhibitors of human Pin1, which inhibitors do not bind to CYP1.
The present inventors thus established a simple and robust method for the identification of compounds fulfilling the above-described needs. Said method for the identification of compounds which selectively and specifically bind to a human peptidyl-prolyl isomerase (PPlase) of the parvulin family comprising the steps of a) incubating a compound of interest with a first mutant yeast strain, wherein said first mutant yeast strain is deficient for an orthologue of a human PPlase of the parvulin family, and wherein the deficiency of said orthologue of said human PPlase of the parvulin family has been rescued by expressing said human PPlase of the parvulin family in said first mutant yeast strain, and b) incubating said compound of interest of step a) with a second mutant yeast strain, wherein said second mutant yeast strain is also deficient for said orthologue of a human PPlase of the parvulin family of step a), and wherein the deficiency of said orthologue of the human PPlase of the parvulin family has been rescued by expressing a human cyclophilin in said second mutant yeast strain, wherein a compound of interest which inhibits the growth of the first mutant yeast strain, but does not inhibit the growth of the second mutant yeast strain is a compound which selectively and specifically binds to a human peptidyl-prolyl isomerase of the parvulin family.
In a preferred embodiment of the invention, the human PPlase of the parvulin family is Pin1. In another preferred embodiment the human cyclophilin is cyclophilin A (CYP1).
Preferably, the yeast strain S. *cerevisiae.* More preferably, the orthologue of a human PPlase of the parvulin family is ESS1. In a most preferred embodiment of the invention, the orthologue of a human PPlase of the parvulin family is an essential gene.

### Description of the figures

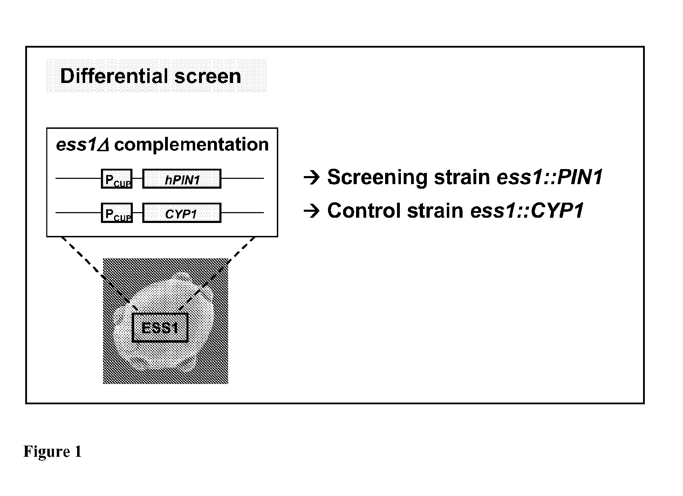

**Figure 1****:** Illustration of the concept of the invention. The assay is based on yeast growth inhibition using the mutant yeast strains *ess1::Pin1* (forward screen) and the corresponding control yeast strain *ess1::CYP1* (counter screen) to eliminate non-specific yeast growth inhibitors. Compounds inhibiting the *ess1::Pin1,* but not the *ess1::CYP1* cell line are considered specific.

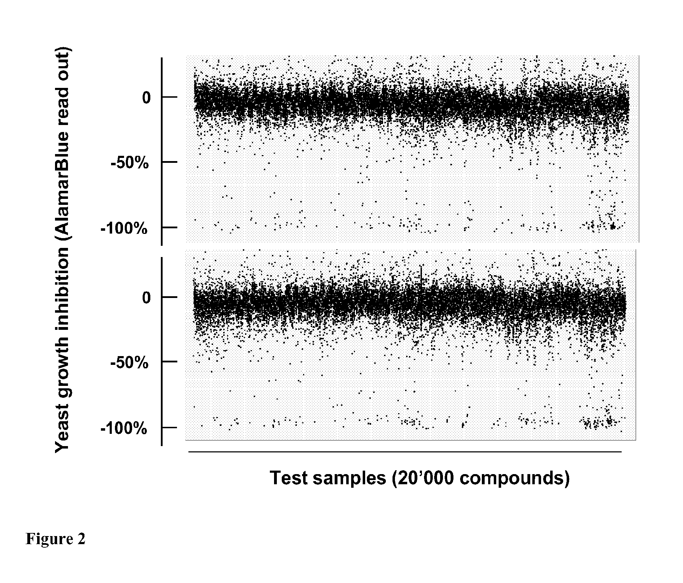

**Figure 2****:** Screening of test samples. The assay described herein was used to screen a library of 20'000 test compounds against *ess1::Pin1* (top panel) and the corresponding control *ess1::CYP1* (bottom panel). Activity of the compounds is measured as yeast growth inhibition using the reporter AlamarBlue. Compounds inhibiting the *ess1::Pin1* (forward screen), but not the *ess1::CYP1* (counter screen) cell line are considered specific.

### Detailed description of the invention

The approach taken by the present inventors has been to elaborate a simple and robust method to find compounds which selectively and specifically bind to a human peptidyl-prolyl isomerase (PPlase) of the parvulin family. Said method comprise the steps of
a) incubating a compound of interest with a first mutant yeast strain, wherein said first mutant yeast strain is deficient for an orthologue of a human PPlase of the parvulin family, and wherein the deficiency of said orthologue of said human PPlase of the parvulin family has been rescued by expressing said human PPlase of the parvulin family in said first mutant yeast strain, and
b) incubating said compound of interest of step a) with a second mutant yeast strain,
wherein said second mutant yeast strain is also deficient for said orthologue of a human PPlase of the parvulin family of step a), and wherein the deficiency of said orthologue of the human PPlase of the parvulin family has been rescued by expressing a human cyclophilin in said second mutant yeast strain,
wherein a compound of interest which inhibits the growth of the first mutant yeast strain, but does not inhibit the growth of the second mutant yeast strain is a compound which selectively and specifically binds to a human peptidyl-prolyl isomerase of the parvulin family.

An exemplary assay of the invention is the Pin1/CYP1 assay illustrated in the examples,
wherein the yeast orthologue of Pin1, ESS1 is replaced by Pin1 and CYP1, alternatively. ESS1, the yeast orthologue of Pin1, regulates confirmation and function of a number of phosphoproteins involved in various cellular processes including cell-cycle control, transcription and splicing regulation, DNA replication checkpoint control, DNA damage response and others.

As explained above, peptidyl-prolyl isomerases (PPlase) are highly conserved during evolution and therefore found in bacteria, yeast, plants, fly and mammals. Three families of structurally unrelated prolyl isomerases are known to date comprising cyclophilins, FKBPs and parvulins.
It will hence be immediately evident to the skilled person that the above system is only exemplary and that any system wherein a yeast orthologue of a gene known to be an orthologue of a PPlas, can be used similarly. Such alternative system include but are not limited to cyclophillins and FK506-binding proteins (FKPBs).

The expression "yeast cell" is used herein to mention unicellular fungi of the phylum *Ascomycota* that reproduce by fission or budding and are capable of fermenting carbohydrates into alcohol and carbon dioxide. Preferably said yeast cells are *Saccharomycetaces* or *Schizossaccharomycefales,* such as *Saccharomyces pombe, Saccharomyces cerevisiae, Candida* or *Pichia.* Most preferably yeast cells of the species *Saccharomyces cerevisiae* are used for manipulation in accordance with the present invention. A genetically stable yeast cell is also referred to as a yeast strain.

A "mutant" refers to an organism which, as a result of either natural processes or mutagenesis techniques including but not limited to site directed mutagenesis, contains one or more mutations (deletions, insertions and/or substitutions) in its genome as compared to the wild-type strain (used for its taxonomic classification), which mutation(s) leads to an inactivation of the target. Preferred embodiments of such mutant according to the present invention are deletion yeast strains, more particularly, deletion strains comprising one or more deletions in one or more genes coding for the target, resulting in the strain lacking a functional gene for the target or a functional target. A functional gene, as used herein refers to a gene which is capable of expressing its natural gene product.

In the context of the present invention, yeast strains "lacking one or more functional genes" are yeast strains in which one or more genes cannot be expressed (no gene product) or which express an aberrant gene product, i.e. a product which is not the natural gene product. This definition also applies *mutatis mutandis* for any non-human organism. Preferably, according to the present invention, such an aberrant gene product, is a gene product which has lost its original function (as present in the wild-type strain). Alternatively such aberrant gene product has a reduced function (less than 50%, less than 75%, or even less than 90%) when compared to the wild type function (enzymatic activity, binding affinity etc.). Yeast cells or strains lacking one or more functional genes can be the result of different factors, such as, but not limited to one or more mutations in the gene itself or in a gene encoding a factor (protein, RNA, dsRNA) which is capable of modifying the expression of said gene, a truncation (caused e.g. by aberrant expression of an enzyme capable of degrading said protein); the complete absence of a gene; the insertion of a transposon; expression of a transgene (resulting in missense, antisense, sense expression) which modifies the expression or the activity of the natural gene product.

The term "inhibition" is used herein to denote the activity of a compound which by interfering with its target, directly or indirectly, influences fundamental processes leading to reduced growth of the treated cells. Reduced growth is defined by the potential of a compound to completely or partially stop or slow down proliferation of a cell. Growth inhibition can be determined by several state of the art techniques monitoring cell number. These include measuring turbidity of a cell culture or using fluorescent, chromophoric or radioactive probes reflecting the number of living cells in a given sample. Growth inhibition of a test compound is determined by a statistically significant reduction in cell number, compared to its corresponding placebo control. A statistically significant growth reduction can be usually determined for a inhibition range of 10% to 100%.

The term "compound" is used herein to denote a chemical structure, a mixture of chemical structures, a peptide, a biological substance such as a macromolecule, or an extract made from biological materials such as bacteria, plants, fungi, or animal (particularly mammalian) cells or tissues. Preferred embodiments are chemical structures and a mixture of chemical structures. According to one aspect of the present invention, methods are provided for identifying compounds capable of influencing, modifying (increasing or decreasing) the phenotypic effect of the target gene or protein in yeast. The influence of a compound on the phenotypic effect observed in yeast cells can be direct, by interacting with the target, or can be indirect, by affecting the expression of the target protein in yeast, or by affecting inhibitors or enzymes which themselves influence the phenotypic effect of the target. Alternatively, the effect of these compounds may be by interacting with proteins or enzymes which directly mediate the phenotypic enact of the target. Such compounds include but are not limited to proteins or polypeptides (such as heat shock proteins receptors, enzymes, ligands, nuclear or cytoplasmic proteins, chaperones, hormones, antigens, growth factors, regulatory proteins, structural proteins) nucleic acids (RNAs, ribozymes, DNAs or cDNAs) or chemical compounds. The screening of compounds from natural or synthetic libraries is also envisaged.

A screening method, as used herein refers to a method for identifying specific compounds. More particularly, the screening method of the present invention allows the identification of compounds based on their ability to selectively and specifically bind to a human peptidyl-prolyl isomerase (PPlase) of the parvulin family.

### Examples

The illustrative assay disclosed herein is based on yeast growth inhibition using the mutant yeast strains *ess1::Pin1* (forward screen) and the corresponding control yeast strain *ess1::CYP1* (counter screen) to eliminate nonspecific yeast growth inhibitors (see figure 1). Compounds inhibiting the *ess1::Pin1,* but not the *ess1::CYP1* cell line are considered specific.

Assay protocol:
Inoculate 2 milliliters YPD medium with the corresponding yeast strains and incubate the cultures over night at 30°C with constant shaking at 170 rpm. The next day, use 250 micro-liters of the over night culture to inoculate 100 milliliters SD medium supplemented with Leucine (10 mg/l), Histidine (20 mg/l), Adenine (20 mg/l) and Tryptophan (20 mg/l). Incubate at 30°C constantly shaking at 170 rpm until an OD600 of 0.8 - 1.0 is reached. Then dilute back the cultures to an OD600 of 0.01 and add 5 µl of these cultures into the 1536 well assay plates containing the test compounds at a final concentration of 20 micro-molar. Further incubate for 16 hours at 30°C in humid atmosphere (95% humidity). After 16 hours add 2.4 micro-liters of 150 mM KPi pH6.4, AlamarBlue 4.5% (v/v) and further incubate for 3 hours at 30°C in 95% humidity. Read plates in a standard plate reader (e.g. Viewlux or EnVision) using an emission wavelength of 560 nano-meters and an excitation wavelength of 590 nano-meters.

After its development, the screening assay of the invention was scaled-down to the 1536 well format and used to test a set of 20'000 compounds at a final compound concentration of 20 µM (figure 2). The screen was found to be highly sensitive (H/L ~7.5) and robust (z' > 0.8) and lead to the identification of compounds with significant selectivity.

### References

Butterfield DA, Abdul HM, Opii W, Newman SF, Joshi G, Ansari MA, Sultana R. (2006). Pin1 in Alzheimer's disease. J Neurochem. 2006 Sep;98(6):1697-706.

Cardenas ME, Zhu D, Heitman J. (1995). Molecular mechanisms of immunosuppression by cyclosporine, FK506, and rapamycin. Curr Opin Nephrol Hypertens. 1995 Nov;4(6):472-7.

Fujimori F, Gunji W, Kikuchi J, Mogi T, Ohashi Y, Makino T, Oyama A, Okuhara K, Uchida T, Murakami Y. (2001). Crosstalk of prolyl isomerases, Pin1/Ess1, and cyclophilin A. Biochem Biophys Res Commun. 2001 Nov 23;289(1):181-90.

Galat A. (2003). Peptidylprolyl cis/trans isomerases (immunophilins): biological diversitytargets--functions. Curr Top Med Chem. 2003;3(12):1315-47.

Lu KP (2004). Pinning down cell signaling, cancer and Alzheimer's disease. Trends Biochem Sci. 2004 Apr;29(4):200-9.

Lu KP, Suizu F, Zhou XZ, Finn G, Lam P, Wulf G. (2006). Targeting carcinogenesis: a role for the prolyl isomerase Pin1? Mol Carcinog. 2006 Jun;45(6):397-402.

Wulf G, Finn G, Suizu F, Lu KP. (2005). Phosphorylation-specific prolyl isomerization: is there an underlying theme? Nat Cell Biol. 2005 May;7(5):435-41.

## Claims

1. A method for the identification of compounds which selectively and specifically bind to a human peptidyl-prolyl isomerase (PPlase) of the parvulin family comprising the steps of
a) incubating a compound of interest with a first mutant yeast strain, wherein said first mutant yeast strain is deficient for an orthologue of a human PPlase of the parvulin family, and wherein the deficiency of said orthologue of said human PPlase of the parvulin family has been rescued by expressing said human PPlase of the parvulin family in said first mutant yeast strain, and
b) incubating said compound of interest of step a) with a second mutant yeast strain,
wherein said second mutant yeast strain is also deficient for said orthologue of a human PPlase of the parvulin family of step a), and wherein the deficiency of said orthologue of the human PPlase of the parvulin family has been rescued by expressing a human cyclophilin in said second mutant yeast strain,
wherein a compound of interest which inhibits the growth of the first mutant yeast strain, but does not inhibit the growth of the second mutant yeast strain is a compound which selectively and specifically binds to a human peptidyl-prolyl isomerase of the parvulin family.

2. The method of claim 1 wherein the human PPlase of the parvulin family is Pin1.

3. The method of claims 1 or 2 wherein the human cyclophilin is cyclophilin A *(CYP1).*

4. The method of any of claims 1 to 3 wherein the yeast strain S. *cerevisiae.*

5. The method of any of claims 1 to 5 wherein the orthologue of a human PPlase of the parvulin family is *ESS1.*

6. The method of any of claims 1 to 5 wherein the orthologue of a human PPlase of the parvulin family is an essential gene.
